# EUROPEAN PATENT SPECIFICATION

(11) **EP 4 159 157 B1**
(45) Date of publication and mention of the grant of the patent: **02.07.2025**
(21) Application number: 21817424.1
(22) Date of filing: 12.05.2021
(51) Int. Cl.: A61C 7/00, A61C 7/08, A61B 5/00

(54) **TRANSPARENT BRACES DESIGN METHOD FOR CREATING TREATMENT PLAN, AND APPARATUS THEREFOR**
VERFAHREN ZUM ENTWURF TRANSPARENTER KLAMMERN ZUR ERSTELLUNG EINES BEHANDLUNGSPLANS UND VORRICHTUNG DAFÜR
PROCÉDÉ DE CONCEPTION D'APPAREIL ORTHODONTIQUE TRANSPARENT POUR LA CRÉATION D'UN PROGRAMME DE SOINS, ET APPAREIL ASSOCIÉ

(30) Priority: 02.06.2020 KR 20200066434
(43) Date of publication of application: 05.04.2023
(73) Proprietor: Osstem Implant Co., Ltd., Gangseo-gu Seoul 07789 (KR)
(72) Inventor: CHOI, Kyoo Ok, Seoul 07789 (KR); JUNG, Ga Young, Bucheon-si Gyeonggi-do 14485 (KR)
(74) Representative: Klunker IP Patentanwälte PartG mbB
(86) International application number: PCT/KR2021/005932
(87) International publication number: WO 2021/246673

(56) References cited:
- KR-A- 20050 082 526
- KR-A- 20190 077 849
- KR-A- 20200 046 843
- KR-B1- 101 138 354
- US-A1- 2017 112 594
- US-A1- 2020 146 775

## Description

### [Technical Field]

The present invention relates to an image analysis and processing technique, and more particularly, to a technique for establishing a dental treatment plan through image analysis. To this effect, the present invention provides a transparent braces design method performed by a transparent braces design apparatus, and the corresponding transparent braces design apparatus.

### [Background Art]

A transparent orthodontic method, which is one of orthodontic treatment methods, is a method of straightening teeth by manufacturing transparent braces, which change from a state of the teeth before an orthodontic treatment to a state of the teeth after the orthodontic treatment, and putting the transparent braces on the teeth. The transparent orthodontic method is rapidly emerging as an orthodontic treatment preferred by patients because this method provides a solution to problems, e.g., a feeling of irritation, mental stress, and a non-aesthetic appearance, that patients may feel when an existing bracket method is used.

However, all-in-one transparent braces may cause another problem. Existing bracket type braces cannot be removed by a patient once attached onto the patient's teeth and thus a technician can conduct desired work completely and quickly, whereas transparent braces can be attached onto or detached from a patient's teeth by the patient and thus the completeness of a treatment is greatly influenced by the patient's degree of cooperation. Unlike the bracket type braces that are individually attached onto each tooth to individually allow movement of each tooth, the transparent braces that are all-in-one type braces do not allow movement of an individual tooth.

A digital method has been introduced as one of the attempts to fix the above problem of transparent braces. When the digital method is used, an appropriate force may be applied to an appropriate position on transparent braces and thus it is expected that teeth can be individually controlled. In addition, a patient's post-treatment condition may be predicted in advance, transparent braces may be manufactured for each stage according to the patient's dental condition or treatment plan until treatment is completed, and appropriate feedback may be given through simple modifications.

However, current technology focuses on individual tooth movement and thus is limited in that an indirect effect of teeth close to a tooth, which is to be moved, on the tooth is not taken into account.

US 2020/0146775 A1, as the closest prior art, discloses systems and methods for treating teeth to correct for malocclusions by receiving a scanned dental model of a subject's dentition, determining a treatment plan having a plurality of incremental movements for repositioning one or more teeth of the subject's dentition, and fabricating one or more aligners correlating to a first subset of the plurality of incremental movements.

### [Disclosure]

### [Technical Problem]

The present invention is directed to a transparent braces design method and apparatus for causing irregular teeth to be accurately straightened without damaging teeth by improving individual tooth movement during establishment of an orthodontic treatment plan to minimize the difference between a patient treatment plan and an actual treatment result.

### [Technical Solution]

Thus, it is an object of the present invention to provide a transparent braces design method performed by a transparent braces design apparatus, and the corresponding transparent braces design apparatus.

The object is achieved by the features of independent claim 1 regarding the method and of independent claim 13 regarding the apparatus. Further embodiments are defined in the respective dependent claims.

The establishing of the treatment plan may include generating a tooth model of the patient, generating a setup model in which the tooth model is moved to a target position according to a tooth movement plan, and generating a braces model for moving the tooth model to the setup model.

The predicting of the treatment result may include quantifying a difference between the tooth model to be moved according to the treatment plan and the transparent braces model when the tooth of the patient overlaps the transparent braces model, providing a color map for representing the quantified difference in a form of identifiable visual information, and predicting an effect of movement of adjacent teeth on the tooth, which is to be moved, using information of the color map.

The quantifying of the difference between the tooth model to be moved according to the treatment plan and the transparent braces model may include quantifying a difference between a tooth model and a braces model for each tooth when the tooth model overlaps the braces model.

The quantifying of the difference between the tooth model to be moved according to the treatment plan and the transparent braces model may include quantifying the difference by measuring a horizontal length of an exposed portion of the tooth model or the transparent braces model, when the tooth model is exposed inside or outside the transparent braces model when the tooth model overlaps the transparent braces model.

The quantifying of the difference between the tooth model to be moved according to the treatment plan and the transparent braces model may include overlapping the tooth model with the transparent braces model, generating a difference region between the tooth model and the transparent braces model when the tooth model overlaps the transparent braces model, and quantifying a size of the difference region between the tooth model and the transparent braces model.

When the quantified difference is a positive (+) value, it may be determined that a force is applied to the tooth by the transparent braces and the force applied to the tooth by the transparent braces increases as the quantified difference increases.

The providing of the color map may include classifying the quantified difference as a preset reference range in advance on the basis of a maximum tooth movement range for each orthodontic stage, identifying a reference range within which a new value falls when the new value is obtained in a state in which classified reference ranges are set to be matched with visual information, and displaying visual information corresponding to the identified reference range.

The predicting of the effect of the movement of adjacent teeth may include predicting, on the basis of the color map, that a collision is likely to occur between teeth during orthodontic treatment when certain visual information is displayed between two adjacent teeth to be moved.

The predicting of the effect of the movement of adjacent teeth may include predicting, based on the color map, that a position of a normal tooth is likely to change during orthodontic treatment when certain visual information is displayed on the normal tooth and an adjacent tooth, which is to be moved, within a preset distance from the normal tooth.

The predicting of the treatment result may include predicting an effect of adjacent teeth for each orthodontic stage from an initial state of the tooth of the patient to a final setup state in which the orthodontic treatment is completed.

The modifying of the treatment plan may include suggesting a direction of treatment by providing information for automatically or manually modifying a part of the treatment plan that is predicted to result in a problem according to the prediction of the treatment result, and establishing a final treatment plan in response to the suggested direction of treatment.

The controller may be further configured to quantify a difference between the tooth model to be moved according to the treatment plan and the transparent braces model when the tooth model of the patient overlaps the transparent braces model, construct a color map for representing the quantified difference in a form of identifiable visual information, and predict an effect of movement of adjacent teeth on the tooth, which is to be moved, using information of the color map.

### [Advantageous Effects]

According to a transparent braces design method and apparatus according to an embodiment, a tooth to be moved can be moved smoothly according to an orthodontic treatment plan without being interfered by adjacent teeth to minimize a difference between a patient treatment plan and an actual treatment result, and thus irregular teeth can be straightened without damaging the teeth to increase a patient's satisfaction and shorten a treatment period, thereby increasing overall treatment efficiency.

A determined treatment plan can be checked by software once more by generally taking into account not only a tooth to be moved but also an effect of the movement of adjacent teeth on the tooth, and a color map can be applied to assist a technician's visual inspection, thereby simplifying a process modification stage.

Because an effect of the movement of adjacent teeth can be predicted in advance during the establishment of an orthodontic treatment plan, a treatment result can be visually checked during treatment without having to check a result of predicting the treatment result, thereby reducing consultation hours, the accuracy of the orthodontic treatment plan can be increased to improve the stability, predictability, accuracy, and convenience of dental treatment, and a target plan between a patient and a technician can be visualized during the establishment of the orthodontic treatment plan, thereby increasing a treatment agreement rate. In addition, a medical team can predict an aesthetic result in advance during the establishment of a treatment plan, and thus can establish the treatment plan in response to a better treatment result and contribute to high-quality treatment.

### [Description of Drawings]

FIG. 1 is a diagram illustrating a configuration of a transparent braces design apparatus according to an embodiment of the present disclosure.
FIG. 2 is a flowchart of a transparent braces design method according to an embodiment of the present disclosure.
FIG. 3 is a diagram illustrating a detailed process of the prediction of a treatment result according to an embodiment of the present disclosure.
FIG. 4 is a diagram illustrating dental images each displaying a difference region between a tooth and braces according to an embodiment of the present disclosure.
FIG. 5 is a dental image displaying a color map according to an embodiment of the present disclosure.

### [Modes of the Invention]

Advantages and features of the present disclosure and methods of achieving the same should become clear from embodiments described in detail below with reference to the accompanying drawings. However, the present disclosure is not limited to the embodiments disclosed below and may be implemented in various different forms. The embodiments herein only make the disclosure of the present disclosure complete and are provided to completely inform those of ordinary skill in the art to which the present disclosure pertains of the scope of the disclosure. The present disclosure is defined only by the scope of the claims. Like components are denoted by like reference numerals throughout the specification.

In describing the embodiments of the present disclosure, when detailed description of a known function or component is determined as having the possibility of unnecessarily obscuring the gist of the present disclosure, the detailed description will be omitted, and the terms used herein are terms defined in consideration of functions in the embodiments of the present disclosure and may vary according to an intention or practice of a user or an operator. Therefore, the terms should be defined on the basis of the content throughout the specification.

Since combinations of blocks of the accompanying block diagram and operations of the accompanying flowchart may be performed by computer program instructions (execution engines) and the computer program instructions may be embedded in processors of general purpose computers, special purpose computers, or other programmable data processing apparatuses, the instructions that are executed by the processors of the computers or other programmable data processing apparatuses generate means for performing functions described in the blocks of the block diagram or the operations of the flowchart.

Since the computer program instructions may be stored in computer usable or computer readable memories capable of being used in computers or other programmable data processing apparatuses to realize functions in particular manners, the instructions stored in the computer usable or computer readable memories can also produce manufacturing items that include instruction means configured to perform the functions described in the blocks of the block diagram or the operations of the flowchart.

In addition, since the computer program instructions may be embedded in the computers or other programmable data processing apparatuses, the instructions, which perform a series of operations in the computers or the other programmable data processing apparatuses to generate computer-executed processes and execute the computers or the other programmable data processing apparatuses, may also provide operations for executing the functions described in the blocks of the block diagram and the operations of the flowchart.

In addition, the blocks or operations may represent portions of modules, segments, or codes including one or more executable instructions for executing specified logical functions, and in some alternative embodiments, the functions described in the blocks or operations may also be performed out of order. For example, two blocks or operations which are sequentially described may also be simultaneously performed, or the blocks or operations may also be performed in reverse order of the corresponding functions as necessary.

Hereinafter, embodiments of the present disclosure will be described in detail with reference to the accompanying drawings. However, the embodiments of the present disclosure described below may be modified into various other forms and the scope of the present disclosure is not limited thereto. Embodiments of the present disclosure are provided to more fully describe the present disclosure to those of ordinary skill in the art.

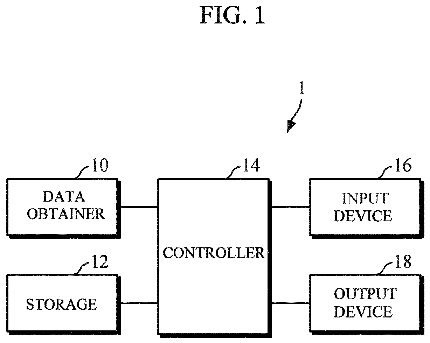

FIG. 1 is a diagram illustrating a configuration of a transparent braces design apparatus according to an embodiment of the present disclosure.

Referring to FIG. 1, the transparent braces design apparatus 1 performs an orthodontic treatment simulation before an orthodontic treatment is actually performed in a dental clinic. The orthodontic treatment simulation is a series of processes of obtaining a patient's clinical data and conducting a simulation by performing diagnosis and analysis using the clinical data to predict a treatment result in advance, under control using software.

The transparent braces design apparatus 1 according to the embodiment may include an electronic device capable of executing digital dental treatment software and a server that communicates with the electronic device through a network. The electronic device may be a computer, a notebook computer, a laptop computer, a tablet PC, a smartphone, a cellular phone, a personal media player (PMP), a personal digital assistant (PDA), or the like.

The present disclosure relates to a technique for manufacturing transparent braces by digital dental treatment software. A digital method has been introduced to manufacture a customized device for a patient by increasing the accuracy of the manufacture of transparent braces. When the digital method is applied, an appropriate force may be applied to an appropriate position on the transparent braces, and thus it is expected that each tooth can be more easily and individually controlled. In addition, a patient's post-treatment condition may be predicted in advance, transparent braces may be manufactured for each stage according to the patient's dental condition or treatment plan until treatment is completed, and appropriate feedback may be given through simple modifications.

Software according to an embodiment is designed to minimize a difference between a patient treatment plan initially designated by a technician and an actual result by taking into account not only individual tooth movement but also an indirect effect of adjacent teeth on a tooth to be moved. In other words, an operation of checking a determined treatment plan by the software once more by taking into account not only a tooth to be moved but also an effect of the movement of adjacent teeth on the tooth prior to the determination of an orthodontic treatment stage is employed unlike in conventional orthodontic treatment methods. In addition, a color map is applied to assist a technician's visual inspection, thereby simplifying a process modification operation.

A configuration of the transparent braces design apparatus 1 having the above-described features will be described with reference to FIG. 1 below.

Referring to FIG. 1, the transparent braces design apparatus 1 according to the embodiment includes a data obtainer 10, a storage 12, a controller 14, an input device 16, and an output device 18.

The data obtainer 10 obtains clinical data from a patient. The clinical data required for dental treatment may be classified into facial data, skeletal data, and dental data, and information of each type of data is as follows.

The facial data is data for identifying the patient's facial structure information, and may be three-dimensional (3D) facial data (front and lateral parts) or two-dimensional (2D) facial data (front part). In this case, in order to represent the teeth when the patient opens his or her mouth, facial data when the patient is smiling or a result of performing matching on the facial data may be used. The 3D facial data is facial data input from a 3D scanner. The 2D facial data may be facial data obtained from a photograph, and may be replaced with data including information equivalent thereto.

The skeletal data is data for identifying the patient's skeletal structure and may be 3D data. 3D skeletal data may be either cephalic data that is in a digital imaging and communications in medicine (DICOM) form or mesh data derived from DICOM, and should include information about facial soft tissue. The 3D skeletal data may be replaced with data including information equivalent thereto. The 3D skeletal data may be computed tomography (CT) data. Information about soft tissue, which is external information of the face, information about the skeletal structure, which is internal information of the face, and information about the mouth may be obtained from the CT data and used for dental consultation.

Additionally, the skeletal data may be 2D skeletal data. The 2D skeletal data may be cephalometric X-ray data, posteroanterior (PA) X-ray data, a lateral cephalogram, or the like.

The dental data is data for identifying the patient's teeth structure information and may be 3D data. 3D dental data may be in a mesh form. The dental data may be dental model scan data obtained by scanning a plaster cast modeled after the patient's oral cavity through a 3D scanner or oral cavity scan data obtained by scanning the inside of the patient's oral cavity using a 3D intra-oral scanner.

The controller 14 predicts a treatment result in advance by performing a simulation through diagnosis and analysis using clinical data while controlling each component during the establishment of an orthodontic treatment plan using software. The controller 14 according to the embodiment designs transparent braces for minimizing the difference between a patient treatment plan and an actual treatment result. During the establishment of the orthodontic treatment plan, it is inevitable that there is a result different from an expected result of a treatment plan initially set by a technician when a surrounding situation is not taken into account in an individual tooth movement plan. The controller 14 predicts an effect of the movement of adjacent teeth to minimize the difference between a planned teeth arrangement and a teeth arrangement after treatment, so that teeth may be moved similar to the treatment plan initially set by the technician.

The controller 14 analyzes clinical data to diagnose the patient's dental condition and establishes an initial treatment plan for tooth movement according to the diagnosed dental condition. In addition, the controller 14 performs a simulation according to the initial treatment plan to predict a treatment result including an effect of adjacent teeth on a tooth to be moved within transparent braces to be manufactured. Furthermore, the initial treatment plan may be modified in response to the predicted effect of the adjacent teeth.

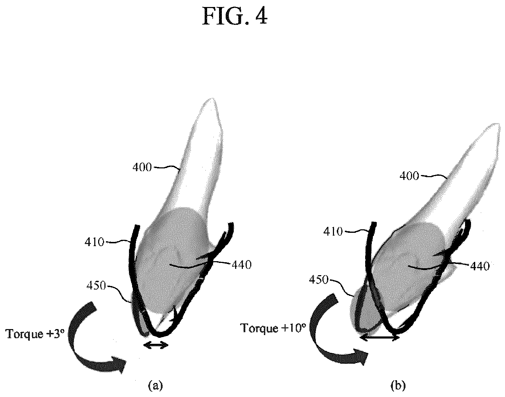

To predict a treatment result, the controller 14 may quantify the difference between a patient's tooth and transparent braces to be worn, when the tooth and the transparent braces overlap, and construct a color map for representing the quantified difference in the form of identifiable visual information. For example, the controller 14 overlaps the tooth with the transparent braces, generates a difference region between the tooth and the transparent braces that overlap, and quantifies a size of the difference area. This embodiment will be described with reference to FIG. 4 below.

The controller 14 may predict an effect of the movement of adjacent teeth on a tooth to be moved, based on information of the color map. For example, on the basis of the color map, the controller 14 predicts that a collision will occur between teeth during orthodontic treatment when certain visual information is displayed between two adjacent teeth to be moved. As another example, on the basis of the color map, the controller 14 predicts that the position of a normal tooth will change during orthodontic treatment when certain visual information is displayed on the normal tooth and an adjacent tooth, which is to be moved, within a preset distance from the normal tooth.

The storage 12 stores various types of data such as information necessary to perform an operation of the transparent braces design apparatus 1 and information generated according to the operation. The storage 12 may provide data to the controller 14 for data analysis by the controller 14.

The output device 18 displays a screen including clinical data and information generated by the controller 14. For example, color map information and dental treatment prediction information may be displayed on an image of the tooth.

The input device 16 receives a user manipulation signal. For example, a manipulation signal for allowing a user to make modifications such as moving or rotating teeth arrangement data displayed on a screen is received through the output device 18. In addition, a reference value for diagnosing each item may be input from a user.

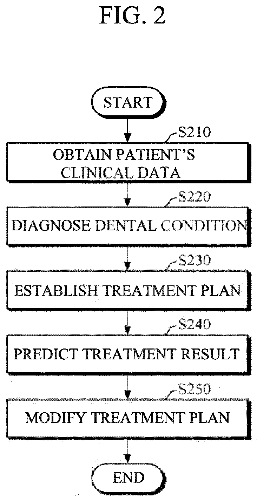

FIG. 2 is a flowchart of a transparent braces design method according to an embodiment of the present disclosure.

Referring to FIG. 2, software obtains a patient's clinical data for digital orthodontic treatment (S210), and diagnoses a dental condition by analyzing the clinical data (S220). In the diagnosing of the dental condition (S220), the software analyzes the patient's clinical data including facial data, skeletal data, and dental data to diagnose the patient's problems.

Next, the software establishes a treatment plan for the movement of a tooth according to the diagnosed dental condition (S230). The establishment of the treatment plan (S230) is an operation of initially setting the treatment plan, in which the software may initially set a tooth movement plan for each stage from an initial state of the patient's tooth to a final setup state in which orthodontic treatment is completed. The establishment of the treatment plan (S230) corresponds to an operation of theoretically setting a plan without taking into account problems that may occur during the movement of the tooth.

In the establishment of the treatment plan (S230), the software may generate a model of the patient's tooth and generate a setup model in which the tooth model is moved to a target position according to the tooth movement plan. In addition, a braces model may be generated to move the teeth model to the setup model.

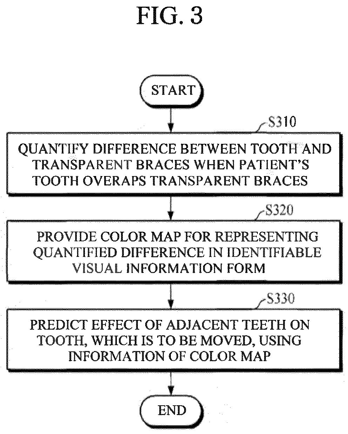

In addition, the software predicts a treatment result, including an effect of adjacent teeth on the tooth to be moved within transparent braces to be manufactured, by performing a simulation according to the treatment plan (S240). A detailed process of the prediction of the treatment result (S240) will be described with reference to FIG. 3 below.

Next, the software modifies the treatment plan in response to the predicted effect of the adjacent teeth (S250). In the modifying of the treatment plan (S250), the software may provide information for automatically or manually modifying a part of the treatment plan that is predicted to result in a problem according to the prediction of the treatment result to suggest a direction of treatment, and establish a final treatment plan in response to the suggested direction of treatment.

In this case, the software resets the direction of treatment by providing the information for modifying a part of the treatment plan, which may interfere with the movement of the tooth, according to the prediction of the treatment result (S240) or by receiving the information from a technician, and applies the suggested direction of treatment to review and designate a final patient treatment plan. For example, the software may automatically change a direction of tooth movement to prevent a collision between teeth or deformation of the tooth or may provide information about the direction of tooth movement. As another example, when a maximum tooth movement range is exceeded and thus a collision between teeth or deformation of the tooth occurs, the maximum tooth movement range may be reduced or information for reducing the maximum tooth movement range may be provided.

FIG. 3 is a diagram illustrating a detailed process of the prediction of a treatment result according to an embodiment of the present disclosure.

Referring to FIGS. 2 and 3, software quantifies the difference between a patient's tooth and transparent braces to be worn when the patient's tooth overlaps the transparent braces (S310). The quantification of the difference between the patient's tooth and the transparent braces (S310) is an initial operation of checking whether a problem such as a collision between teeth or a change of the position of a normal tooth occurs when transparent braces to be manufactured based on a teeth model planned for each stage is applied to actual orthodontic treatment. Transparent braces to be actually manufactured may be represented on an image of the tooth using a braces model. In this case, software may quantify the difference between the tooth model and the braces model for each of the teeth when the tooth model overlaps the braces model.

In the quantification of the difference between the patient's tooth and the transparent braces (S310), when the tooth overlaps the transparent braces to be worn, the software may measure a horizontal length of either an exposed portion of the tooth or the transparent braces to quantify the difference, when the tooth is exposed inside or outside the transparent braces. In the quantification of the difference between the patient's tooth and the transparent braces (S310), it should be understood that when the quantified difference is a positive (+) value, a force is applied to the tooth by the transparent braces, and the force applied to the tooth by the transparent braces may increase as the quantified value increases. When the quantified difference is a negative (-) value, a space to which the tooth to be moved is marked.

Thereafter, the software provides a color map on which the quantified difference is displayed in the form of identifiable visual information (S320). To this end, the software classifies the quantified difference as a preset reference range in advance on the basis of a maximum tooth movement range for each orthodontic stage, and sets each classified reference range to be matched with visual information. When a new value is obtained, a reference range within which the new value falls among previously classified reference ranges is identified, and visual information corresponding to the identified reference range may be displayed.

Thereafter, the software predicts an effect of the movement of adjacent teeth on the tooth, which is to be moved, using color map information (S330). In the prediction of the effect of the movement of adjacent teeth (S330), on the basis of a color map, the software may predict that a collision will occur between teeth during orthodontic treatment when certain visual information is displayed between two adjacent teeth to be moved. As another example, based on the color map, it may be predicted that the position of a normal tooth will change during orthodontic treatment, when certain visual information is displayed on the normal tooth and an adjacent tooth, which is to be moved, within a preset distance from the normal tooth.

FIG. 4 is a diagram illustrating dental images each displaying a difference region between a tooth and braces according to an embodiment of the present disclosure. More specifically, FIG. 4A illustrates an example in which a difference between a tooth and braces is small, and FIG. 4B illustrates an example in which a difference between the tooth and the braces is large.

Referring to FIG. 4, software overlaps a patient's tooth 400 with braces 410. In this case, an overlap region 440 is shown in FIG. 4. The overlap region 440 is an overlapping portion between the tooth 400 and the braces 410. When the tooth 400 and the braces 410 overlap, there may a difference between the tooth 400 and the braces 410, and the software generates a difference region 450 between the tooth 400 and the braces 410. The difference region 450 is a pressed portion of the tooth 400 when a patient wears the braces 410.

As shown in FIG. 4A, when the overlap region 440 between the tooth 400 and the braces 410 increases, the difference region 450 decreases. This means that a force applied to the tooth 400 by the braces 410 is small. In contrast, as shown in FIG. 4B, when the overlap between the tooth 400 and the braces 410 is small, the overlap region 440 decreases and the difference region 450 increases. This means that the force applied to the tooth 400 by the braces 410 is large. The software may express the difference between the tooth 400 and the braces 410 in the form of identifiable visual information according to the magnitude of the force applied to the tooth 400 by the braces 410.

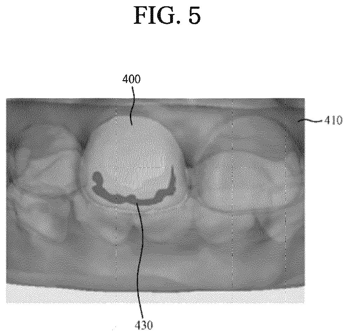

FIG. 5 is a diagram illustrating a dental image displaying a color map according to an embodiment of the present disclosure.

Referring to FIG. 5, a color map 430 is generated by classifying a value obtained by quantifying the difference between a patient's tooth 400 and transparent braces 410 to be worn as a certain range on the basis of a maximum tooth movement range for each stage and thereafter designating a color. For the color map 430, a single color or graduated colors are designated according to a user's taste, and generally, the larger the value, the more red, and the smaller the value, the more blue. However, this is only an example for helping understanding of the present disclosure and embodiments are not limited thereto.

A technician can visually predict how the movement of an individual tooth will proceed through the generated color map 430. For example, it can be predicted that a collision will occur when a part of a color (e.g., reddish color) corresponding to a large value is close between two teeth. As another example, it can be predicted that the position of a normal tooth will change when an adjacent tooth that is not appropriately spaced from the normal tooth has a red part.

## Claims

1. A transparent braces design method performed by a transparent braces design apparatus (1), the method comprising:
(S210) obtaining clinical data of a patient for a digital orthodontic treatment;
(S220) analyzing the clinical data to diagnose a dental condition:
(S230) establishing a treatment plan for tooth movement according to a result of diagnosing the dental condition;
(S240) predicting a treatment result by performing a simulation according to the treatment plan, the treatment result including an effect of adjacent teeth on a tooth to be moved within transparent braces to be manufactured; and
(S250) modifying the treatment plan to reflect the predicted effect of adjacent teeth,
**characterized in that** the (S240) predicting the treatment result comprises predicting a collision between teeth during orthodontic treatment on the basis of a color map for representing the difference value between a tooth model to be moved according to the treatment plan and a transparent braces model.

2. The method of claim 1, wherein the establishing of the treatment plan comprises:
generating a tooth model of the patient;
generating a setup model in which the tooth model is moved to a target position according to a tooth movement plan; and
generating a braces model for moving the tooth model to the setup model.

3. The method of claim 1, wherein the predicting of the treatment result comprises:
(S310) quantifying a difference between the tooth model to be moved according to the treatment plan and the transparent braces model when the tooth of the patient overlaps the transparent braces model;
(S320) providing a color map for representing the quantified difference in a form of identifiable visual information; and
(S330) predicting an effect of movement of adjacent teeth on the tooth, which is to be moved, using information of the color map.

4. The method of claim 3, wherein the (S310) quantifying of the difference between the tooth model to be moved according to the treatment plan and the transparent braces model comprises quantifying a difference between a tooth model and a braces model for each tooth when the tooth model overlaps the braces model.

5. The method of claim 3, wherein the (S310) quantifying of the difference between the tooth model to be moved according to the treatment plan and the transparent braces model comprises quantifying the difference by measuring a horizontal length of an exposed portion of the tooth model or the transparent braces model, when the tooth model is exposed inside or outside the transparent braces model when the tooth model overlaps the transparent braces model.

6. The method of claim 3, wherein the (S310) quantifying of the difference between the tooth model to be moved according to the treatment plan and the transparent braces model comprises:
overlapping the tooth model with the transparent braces model;
generating a difference region between the tooth model and the transparent braces model when the tooth model overlaps the transparent braces model; and
quantifying a size of the difference region between the tooth model to be moved according to the treatment plan and the transparent braces model.

7. The method of claim 3, wherein, when the (S320) quantified difference is a positive (+) value, it is determined that a force is applied to the tooth by the transparent braces, and the force applied to the tooth by the transparent braces increases as the quantified difference increases.

8. The method of claim 3, wherein the (S320) providing of the color map comprises:
classifying the quantified difference as a preset reference range in advance on the basis of a maximum tooth movement range for each orthodontic stage;
identifying a reference range within which a new value falls when the new value is obtained in a state in which classified reference ranges are set to be matched with visual information; and
displaying visual information corresponding to the identified reference range.

9. The method of claim 3, wherein the (S330) predicting of the effect of the movement of adjacent teeth comprises predicting, based on the color map, that a collision is likely to occur between teeth during orthodontic treatment when certain visual information is displayed between two adjacent teeth to be moved.

10. The method of claim 3, wherein the (S330) predicting of the effect of the movement of adjacent teeth comprises predicting, based on the color map, that a position of a normal tooth is likely to change during orthodontic treatment when certain visual information is displayed on the normal tooth and an adjacent tooth, which is to be moved, within a preset distance from the normal tooth.

11. The method of claim 1, wherein the (S240) predicting of the treatment result comprises predicting an effect of adjacent teeth for each orthodontic stage from an initial state of the tooth of the patient to a final setup state in which the orthodontic treatment is completed.

12. The method of claim 1, wherein the (S250) modifying of the treatment plan comprises:
suggesting a direction of treatment by providing information for automatically or manually modifying a part of the treatment plan that is predicted to result in a problem according to the prediction of the treatment result; and
establishing a final treatment plan to reflect the suggested direction of treatment.

13. A transparent braces design apparatus (1) comprising:
a data obtainer (10) configured to obtain clinical data of a patient for a digital orthodontic purpose;
a controller (14) configured to analyze the clinical data to diagnose a dental condition, establish a treatment plan according to the dental condition, predict a treatment result by performing a simulation according to the treatment plan, and modify the treatment plan to reflect an effect of adjacent teeth on a tooth to be moved within transparent braces to be manufactured, the treatment result including the effect of the adjacent teeth; and
an output device (16) configured to display a screen according to operation of the controller,
**characterized in that** the controller (14) is configured to predict a collision between teeth during orthodontic treatment on the basis of a color map for representing the difference value between a tooth model to be moved according to the treatment plan and a transparent braces model.

14. The apparatus (1) of claim 13, wherein the controller (14) is further configured to:
quantify a difference between the tooth model to be moved according to the treatment plan and the transparent braces model when the tooth model of the patient overlaps the transparent braces model;
construct a color map for representing the quantified difference in a form of identifiable visual information; and
predict an effect of movement of adjacent teeth on the tooth, which is to be moved, using information of the color map.

## Patentansprüche

1. Verfahren zum Gestalten von transparenten Zahnspangen, das von einer Vorrichtung (1) zum Gestalten von transparenten Zahnspangen ausgeführt wird, wobei das Verfahren aufweist:
(S210) Erhalten klinischer Daten eines Patienten für eine digitale kieferorthopädische Behandlung;
(S220) Analysieren der klinischen Daten, um einen Zahnzustand zu diagnostizieren;
(S230) Erstellen eines Behandlungsplans für eine Zahnbewegung gemäß einem Ergebnis der Diagnose des Zahnzustands;
(S240) Prognostizieren eines Behandlungsergebnisses durch Ausführen einer Simulation gemäß dem Behandlungsplan, wobei das Behandlungsergebnis eine Wirkung benachbarter Zähne auf einen zu bewegenden Zahn innerhalb herzustellender transparenter Zahnspangen aufweist; und
(S250) Modifizieren des Behandlungsplans, um den prognostizierten Effekt benachbarter Zähne widerzuspiegeln,
**dadurch gekennzeichnet, dass** das (S240) Prognostizieren des Behandlungsergebnisses das Prognostizieren einer Kollision zwischen Zähnen während einer kieferorthopädischen Behandlung auf der Grundlage einer Farbkarte zum Darstellen des Differenzwerts zwischen einem gemäß dem Behandlungsplan zu bewegenden Zahnmodell und einem transparenten Zahnspangenmodell aufweist.

2. Verfahren nach Anspruch 1, wobei das Erstellen des Behandlungsplans aufweist:
Erzeugen eines Zahnmodells des Patienten;
Erzeugen eines Aufstellungsmodells, in dem das Zahnmodell gemäß einem Zahnbewegungsplan zu einer Zielposition bewegt wird; und
Erzeugen eines Zahnspangenmodells zum Bewegen des Zahnmodells zu dem Aufstellungsmodell.

3. Verfahren nach Anspruch 1, wobei das Prognostizieren des Behandlungsergebnisses aufweist:
(S310) Quantifizieren einer Differenz zwischen dem Zahnmodell, das gemäß dem Behandlungsplan bewegt werden soll, und dem transparenten Zahnspangenmodell, wenn der Zahn des Patienten das transparente Zahnspangenmodell überlappt;
(S320) Bereitstellen einer Farbkarte zum Darstellen der quantifizierten Differenz in Form von identifizierbarer visueller Information; und
(S330) Prognostizieren einer Auswirkung der Bewegung benachbarter Zähne auf den Zahn, der bewegt werden soll, unter Verwendung von Information der Farbkarte.

4. Verfahren nach Anspruch 3, wobei das (S310) Quantifizieren des Unterschieds zwischen dem Zahnmodell, das gemäß dem Behandlungsplan bewegt werden soll, und dem transparenten Zahnspangenmodell das Quantifizieren eines Unterschieds zwischen einem Zahnmodell und einem Zahnspangenmodell für jeden Zahn aufweist, wenn das Zahnmodell das Zahnspangenmodell überlappt.

5. Verfahren nach Anspruch 3, wobei das (S310) Quantifizieren der Differenz zwischen dem Zahnmodell, das gemäß dem Behandlungsplan bewegt werden soll, und dem transparenten Zahnspangenmodell die Quantifizierung der Differenz durch Messen einer horizontalen Länge eines freiliegenden Abschnitts des Zahnmodells oder des transparenten Zahnspangenmodells aufweist, wenn das Zahnmodell innerhalb oder außerhalb des transparenten Zahnspangenmodells freiliegt, wenn das Zahnmodell das transparente Zahnspangenmodell überlappt.

6. Verfahren nach Anspruch 3, wobei das (S310) Quantifizierung der Differenz zwischen dem gemäß dem Behandlungsplan zu bewegenden Zahnmodell und dem transparenten Zahnspangenmodell aufweist:
Überlappen des Zahnmodells mit dem transparenten Zahnspangenmodell;
Erzeugen eines Differenzbereichs zwischen dem Zahnmodell und dem transparenten Zahnspangenmodell, wenn das Zahnmodell das transparente Zahnspangenmodell überlappt; und
Quantifizieren einer Größe des Differenzbereichs zwischen dem Zahnmodell, das gemäß dem Behandlungsplan bewegt werden soll, und dem transparenten Zahnspangenmodell.

7. Verfahren nach Anspruch 3, wobei, wenn die (S320) quantifizierte Differenz ein positiver (+) Wert ist, bestimmt wird, dass eine Kraft durch die transparente Zahnspange auf den Zahn ausgeübt wird, und die durch die transparente Zahnspange auf den Zahn ausgeübte Kraft zunimmt, wenn die quantifizierte Differenz zunimmt.

8. Verfahren nach Anspruch 3, wobei das Bereitstellen der Farbkarte (S320) aufweist:
Klassifizieren der quantifizierten Differenz als einen voreingestellten Referenzbereich im Voraus auf der Grundlage eines maximalen Zahnbewegungsbereichs für jede kieferorthopädische Stufe;
Identifizieren eines Referenzbereichs, in den ein neuer Wert fällt, wenn der neue Wert in einem Zustand erhalten wird, in dem klassifizierte Referenzbereiche so eingestellt sind, dass sie mit visuellen Informationen übereinstimmen; und
Anzeige visueller Informationen, die dem identifizierten Referenzbereich entsprechen.

9. Verfahren nach Anspruch 3, wobei das (S330) Prognostizieren der Auswirkung der Bewegung benachbarter Zähne die Prognose auf der Grundlage der Farbkarte aufweist, dass während der kieferorthopädischen Behandlung wahrscheinlich eine Kollision zwischen Zähnen auftritt, wenn bestimmte visuelle Informationen zwischen zwei benachbarten, zu bewegenden Zähnen angezeigt werden.

10. Verfahren nach Anspruch 3, wobei das (S330) Prognostizieren der Auswirkung der Bewegung benachbarter Zähne aufweist: Prognostizieren, basierend auf der Farbkarte, dass sich die Position eines normalen Zahns während der kieferorthopädischen Behandlung wahrscheinlich ändert, wenn bestimmte visuelle Informationen auf dem normalen Zahn und einem benachbarten Zahn, der bewegt werden soll, innerhalb eines vorgegebenen Abstands von dem normalen Zahn angezeigt werden.

11. Verfahren nach Anspruch 1, wobei das (S240) Prognostizieren des Behandlungsergebnisses das Vorhersagen einer Wirkung benachbarter Zähne für jede kieferorthopädische Phase von einem initialen Zustand des Zahns des Patienten bis zu einem endgültigen Aufstellungszustand, in dem die kieferorthopädische Behandlung abgeschlossen ist, aufweist.

12. Verfahren nach Anspruch 1, wobei das (S250) Modifizieren des Behandlungsplans aufweist:
Vorschlagen einer Behandlungsrichtung durch Bereitstellen von Informationen zum automatischen oder manuellen Modifizieren eines Teils des Behandlungsplans, von dem vorhergesagt wird, dass er gemäß der Vorhersage des Behandlungsergebnisses zu einem Problem führt; und
Erstellen eines endgültigen Behandlungsplans, der die vorgeschlagene Behandlungsrichtung widerspiegelt.

13. Eine Vorrichtung (1) zum Gestalten transparenter Zahnspangen, aufweisend:
einen Datenerheber (10), der konfiguriert ist, klinische Daten eines Patienten für einen digitalen kieferorthopädischen Zweck zu erheben;
eine Steuerung (14), die konfiguriert ist, die klinischen Daten zu analysieren, um einen Zahnzustand zu diagnostizieren, einen Behandlungsplan gemäß dem Zahnzustand zu erstellen, ein Behandlungsergebnis durch Ausführen einer Simulation gemäß dem Behandlungsplan zu prognostizieren und den Behandlungsplan zu modifizieren, um eine Wirkung benachbarter Zähne auf einen Zahn, der innerhalb herzustellender transparenter Zahnspangen bewegt werden soll, zu berücksichtigen, wobei das Behandlungsergebnis die Wirkung der benachbarten Zähne aufweist; und
eine Ausgangseinrichtung (16), die konfiguriert ist, einen Anzeigebildschirm gemäß dem Betrieb der Steuerung anzuzeigen,
**dadurch gekennzeichnet, dass** die Steuerung (14) konfiguriert ist, eine Kollision zwischen Zähnen während einer kieferorthopädischen Behandlung auf der Grundlage einer Farbkarte zur Darstellung des Differenzwerts zwischen einem gemäß dem Behandlungsplan zu bewegenden Zahnmodell und einem transparenten Zahnspangenmodell zu prognostizieren.

14. Vorrichtung (1) nach Anspruch 13, wobei die Steuerung (14) weiter konfiguriert ist, um:
eine Differenz zwischen dem Zahnmodell, das gemäß dem Behandlungsplan bewegt werden soll, und dem transparenten Zahnspangenmodell zu quantifizieren, wenn das Zahnmodell des Patienten das transparente Zahnspangenmodell überlappt;
eine Farbkarte zu erstellen, um die quantifizierte Differenz in Form von identifizierbaren visuellen Informationen darzustellen; und
die Auswirkungen der Bewegung benachbarter Zähne auf den zu bewegenden Zahn anhand der Informationen aus der Farbkarte zu prognostizieren.

## Revendications

1. Procédé de conception d'appareillage orthodontique transparent réalisé par un appareil de conception d'appareillage orthodontique transparent (1), le procédé comprenant les étapes suivantes :
(S210) obtenir des données cliniques d'un patient pour un traitement orthodontique numérique ;
(S220) analyser les données cliniques pour diagnostiquer un état dentaire ;
(S230) établir un plan de traitement pour un déplacement dentaire conformément à un résultat de diagnostic sur l'état dentaire ;
(S240) prédire un résultat de traitement en réalisant une simulation conformément au plan de traitement, le résultat de traitement incluant un effet de dents adjacentes sur une dent à déplacer dans les limites de l'appareillage orthodontique transparent à fabriquer, et
(S250) modifier le plan de traitement pour refléter l'effet prédit de dents adjacentes,
**caractérisé en ce que** l'étape (240) pour prédire le résultat de traitement comprend la prédiction d'une collision entre des dents durant le traitement orthodontique sur la base d'une carte de couleurs pour représenter la valeur de différence entre un modèle de dent à déplacer conformément au plan de traitement et un modèle d'appareillage orthodontique transparent.

2. Procédé selon la revendication 1, dans lequel l'étape pour établir le plan de traitement comprend les étapes suivantes :
générer un modèle de dent du patient ;
générer un modèle de réglage, où le modèle de dent est déplacé vers une position cible conformément à un plan de déplacement de dent, et
générer un modèle d'appareillage orthodontique pour déplacer le modèle de dent vers le modèle de réglage.

3. Procédé selon la revendication 1, dans lequel l'étape pour prédire le résultat de traitement comprend les étapes suivantes :
(S310) quantifier une différence entre le modèle de dent à déplacer conformément au plan de traitement et le modèle d'appareillage orthodontique transparent lorsque la dent du patient chevauche le modèle d'appareillage orthodontique transparent ;
(S320) fournir une carte de couleurs pour représenter la différence quantifiée sous une forme d'informations visuelles identifiables, et
(S330) prédire un effet de déplacement de dents adjacentes sur la dent, laquelle doit être déplacée, à l'aide des informations de la carte de couleurs.

4. Procédé selon la revendication 3, dans lequel l'étape (S310) pour quantifier la différence entre le modèle de dent à déplacer conformément au plan de traitement et le modèle d'appareillage orthodontique comprend l'étape pour quantifier une différence entre un modèle de dent et un modèle d'appareillage orthodontique pour chaque dent lorsque le modèle de dent chevauche le modèle d'appareillage orthodontique.

5. Procédé selon la revendication 3, dans lequel l'étape (S310) pour quantifier la différence entre le modèle de dent à déplacer conformément au plan de traitement et le modèle d'appareillage orthodontique transparent comprend l'étape pour quantifier la différence en mesurant une longueur horizontale d'une portion exposée du modèle de dent ou du modèle d'appareillage orthodontique transparent, lorsque le modèle de dent est exposé dans ou hors de l'appareillage orthodontique transparent ou lorsque le modèle de dent chevauche le modèle d'appareillage orthodontique transparent.

6. Procédé selon la revendication 3, dans lequel l'étape (S310) pour quantifier la différence entre le modèle de dent à déplacer conformément au plan de traitement et le modèle d'appareillage orthodontique transparent comprend les étapes suivantes :
faire chevaucher le modèle de dent avec le modèle d'appareillage orthodontique transparent ;
générer une région de différence entre le modèle de dent et le modèle d'appareillage orthodontique transparent lorsque le modèle de dent chevauche le modèle d'appareillage orthodontique transparent, et
quantifier une taille de la région de différence entre le modèle de dent à déplacer conformément au plan de traitement et le modèle d'appareillage orthodontique transparent.

7. Procédé selon la revendication 3, dans lequel lorsque la différence quantifiée (S320) est une valeur positive (+), il est déterminé qu'une force est appliquée à la dent par l'appareil orthodontique transparent, et la force appliquée à la dent par l'appareillage orthodontique transparent augmente à mesure que la différence quantifiée augmente.

8. Procédé selon la revendication 3, dans lequel l'étape (S320) pour fournir la carte de couleurs comprend les étapes suivantes :
classer la différence quantifiée comme plage de référence préréglée à l'avance sur la base d'une plage de déplacement de dent maximale pour chaque stade orthodontique ;
identifier une plage de référence dans les limites dans laquelle une nouvelle valeur tombe lorsque la nouvelle valeur est obtenue dans un état où des plages de référence classées sont réglées pour être mises en concordance avec des informations visuelles, et
afficher des informations visuelles correspondant à la plage de référence identifiée.

9. Procédé selon la revendication 3, dans lequel l'étape (S330) pour prédire l'effet du déplacement de dents adjacentes comprend l'étape pour prédire, sur la base de la carte de couleurs, la probabilité d'occurrence d'une collision entre des dents durant un traitement orthodontique lorsque certaines informations visuelles sont affichées entre deux dents adjacentes à déplacer.

10. Procédé selon la revendication 3, dans lequel l'étape (S330) pour prédire l'effet du déplacement de dents adjacentes comprend l'étape pour prédire, sur la base de la carte de couleurs, la probabilité d'une modification d'une position d'une dent normale durant un traitement orthodontique lorsque certaines informations visuelles sont affichées sur la dent normale et une dent adjacente, laquelle doit être déplacée, dans les limites d'une distance préréglée par rapport à la dent normale.

11. Procédé selon la revendication 1, dans lequel l'étape (S240) pour prédire le résultat de traitement comprend l'étape pour prédire un effet de dents adjacentes pour chaque stade orthodontique à partir d'un état initial de la dent du patient jusqu'à un état de réglage final, où le traitement orthodontique est terminé.

12. Procédé selon la revendication 1, dans lequel l'étape (250) pour modifier le plan de traitement comprend l'étape suivante :
suggérer une direction de traitement en fournissant des informations pour modifier automatiquement ou manuellement une partie du plan de traitement, laquelle est prédite comme résultant en un problème conformément à la prédiction du résultat de traitement, et
établir un plan de traitement final pour refléter la direction suggérée de traitement.

13. Appareil de conception d'appareillage orthodontique transparent (1), comprenant les éléments suivants :
un moyen d'obtention de données (10) configuré pour obtenir des données cliniques d'un patient pour un traitement orthodontique numérique ;
un contrôleur (14) configuré pour analyser les données cliniques pour diagnostiquer un état dentaire, établir un plan de traitement conformément à un état dentaire, prédire un résultat de traitement en réalisant une simulation conformément au plan de traitement, et modifier le plan de traitement pour refléter un effet de dents adjacentes sur une dent à déplacer dans les limites de l'appareillage orthodontique transparent à fabriquer, le résultat de traitement incluant l'effet des dents adjacentes, et
un dispositif de sortie (16) configuré pour afficher un écran conformément au fonctionnement du contrôleur,
**caractérisé en ce que** le contrôleur (14) est configuré pour prédire une collision entre des dents durant un traitement orthodontique sur la base d'une carte de couleurs pour représenter la valeur de différence entre un modèle de dent à déplacer conformément au plan de traitement et un modèle d'appareillage orthodontique transparent.

14. Appareil (1) selon la revendication 13, dans lequel le contrôleur (14) est en outre configuré pour :
quantifier une différence entre le modèle de dent à déplacer conformément au plan de traitement et le modèle d'appareillage orthodontique transparent lorsque le modèle de dent du patient chevauche le modèle d'appareillage orthodontique transparent ;
construire une carte de couleurs pour représenter la différence quantifiée sous une forme d'informations visuelles identifiables, et
prédire un effet de déplacement de dents adjacentes sur la dent, laquelle doit être déplacée, à l'aide des informations de la carte de couleurs.
